# FASCICULE DE BREVET EUROPEEN

(11) **EP 1 584 328 B1**
(45) Date de publication et mention de la délivrance du brevet: **19.07.2017**
(21) Numéro de dépôt: 05300249.9
(22) Date de dépôt: 01.04.2005
(51) Int. Cl.: A61K 8/46, A61K 8/49, A61K 8/55, A61K 8/23, A61Q 5/04

(54) **Procédé de traitement capillaire et utilisation dudit procédé**
Haarbehandlungsverfahren und dessen Einsatz
Hair treatment method and its use

(30) Priorité: 02.04.2004 FR 0450666
(43) Date de publication de la demande: 12.10.2005
(73) Titulaire: L'Oréal, 75008 Paris (FR)
(72) Inventeur: Fondin, Thomas, 95150 Taverny (FR); Sabbagh, Anne, 92500 Rueil Malmaison (FR)
(74) Mandataire: Caillet, Isabelle

(56) Documents cités:
- EP-A- 0 299 764
- CH-A- 353 491
- US-A- 3 256 154
- PATENT ABSTRACTS OF JAPAN vol. 2003, no. 04, 2 avril 2003 (2003-04-02) & JP 2002 356408 A (MILBON CO LTD), 13 décembre 2002 (2002-12-13) -& JP 2002 356408 A 13 décembre 2002 (2002-12-13)
- PATENT ABSTRACTS OF JAPAN vol. 2003, no. 12, 5 décembre 2003 (2003-12-05) & JP 2004 026770 A (MILBON CO LTD), 29 janvier 2004 (2004-01-29) -& JP 2004 026770 A 29 janvier 2004 (2004-01-29)
- DATABASE WPI Section Ch, Week 200107 Derwent Publications Ltd., London, GB; Class D21, AN 2001-052903 XP002307563 & JP 2000 256146 A (MIRUBON KK) 19 septembre 2000 (2000-09-19) -& JP 2000 256146 A 19 septembre 2000 (2000-09-19)
- DATABASE WPI Section Ch, Week 200170 Derwent Publications Ltd., London, GB; Class D21, AN 2001-609747 XP002307747 & JP 2001 213741 A (HOYU KK) 7 août 2001 (2001-08-07) -& JP 2001 213741 A 7 août 2001 (2001-08-07)

## Description

La présente invention se rapporte à un procédé de traitement des fibres capillaires, ainsi qu'à l'utilisation dudit procédé.

Il est usuel, pour obtenir la déformation permanente des cheveux, de réaliser dans un premier temps l'ouverture des liaisons disulfures de la kératine (cystine) à l'aide d'une composition contenant un agent réducteur, puis après avoir de préférence rincé les cheveux, à reconstituer dans un second temps lesdites liaisons disulfures en appliquant sur les cheveux lissés ou préalablement mis sous tension par des moyens adéquats tels que les bigoudis, une composition oxydante encore appelée fixateur de façon à donner à la chevelure la forme recherchée. Cette technique permet indifféremment de réaliser soit l'ondulation des cheveux, soit leur défrisage, leur crêpage ou leur lissage.

Les compositions réductrices utilisables pour la mise en oeuvre de la première étape de ces procédés contiennent généralement des composés thiolés, tels que l'acide thioglycolique, la cystéine, la cystéamine, l'acide thiolactique et le monothioglycolate de glycérol.

Cependant, une telle technique ne donne pas entièrement satisfaction. En effet, cette technique est très efficace pour modifier la forme des cheveux, mais très dégradante pour les fibres capillaires.

Il a en outre été proposé d'élever la température des cheveux, entre l'étape de réduction et l'étape de fixation, au moyen d'un fer chauffant.

Ainsi, la demande de brevet JP 2000 256146 décrit un procédé de déformation permanente des cheveux comprenant l'application d'une composition cosmétique contenant 2 à 11% d'agents réducteurs et de 0,2 à 4% de dithioglycolate de diammonium. L'application de la composition réductrice est suivie du passage d'un fer chauffant entre 60 et 220°C.

Cependant, lorsque l'on utilise un fer chauffant, la forme obtenue est irréversible. Le contraste entre les parties traitées et les racines est alors important au moment de la repousse des cheveux.

Enfin, si le traitement est effectué sur des cheveux colorés, on constate très souvent une forte altération de la couleur due au traitement.

Le but de la présente invention est donc de fournir un procédé de traitement des fibres capillaires qui remédie aux inconvénients de l'art antérieur.

En particulier, la présente invention a pour but de fournir un procédé de traitement des fibres capillaires qui permet de modifier le comportement de la fibre capillaire en limitant son altération, de maîtriser le volume des cheveux et d'améliorer les performances cosmétiques des cheveux, notamment la douceur, la brillance et le démêlage en respectant mieux la couleur des cheveux teints.

Ledit procédé doit permettre en outre aux cheveux de garder un aspect naturel, ce qui limite l'effet racines, c'est-à-dire le contraste entre les parties traitées et les racines.

Enfin, l'invention a pour but de réduire la durée du traitement des fibres capillaires et d'obtenir des effets durables.

La Demanderesse a trouvé qu'il était possible de remédier aux inconvénients de l'art antérieur et de remplir les objectifs précités, en mettant en oeuvre un procédé de traitement des fibres capillaires comprenant une étape d'application sur les fibres capillaires d'une composition réductrice contenant au moins un agent réducteur particulier, puis une étape d'élévation de la température des fibres capillaires, au moyen d'un fer chauffant, à une température au moins égale à 60°C, l'élévation de la température étant effectuée avant ou après le rinçage éventuel des fibres capillaires.

L'invention a pour objet un procédé de traitement des fibres capillaires comprenant les étapes suivantes :
- application sur les fibres capillaires d'une composition réductrice contenant au moins un agent réducteur, le ou lesdits agents réducteurs étant choisis permis les sulfites ou les bisulfites, ladite composition réductrice ne contenant pas de céramides.
- élévation de la température des fibres capillaires, au moyen d'un fer chauffant, à une température au moins égale à 60°C, l'élévation de la température étant effectuée avant ou après le rinçage éventuel des fibres capillaires.

De préférence, la composition réductrice est appliquée sur des fibres capillaires humides et propres.

A titre de sulfites et de bisulfites utilisables dans la composition selon l'invention, on peut citer les sulfites ou bisulfites des métaux alcalins ou alcalino-terreux ou d'ammonium et en particulier le sulfite ou le bisulfite de sodium, de potassium ou les sulfites ou bisulfites d'alcanolamines tels que le sulfite ou bisulfite de monoéthanolamine.

Lorsque la composition réductrice contient un ou plusieurs sulfites ou bisulfites, avantageusement elle ne contient pas d'acide dithiodiglycolique ou l'un de ses sels.

Le ou les agents réducteurs représentent généralement 0,1 à 30%, de préférence de 0,5 à 20%, mieux de 1 à 10%, en poids par rapport au poids total de la composition réductrice.

Le pH de la composition réductrice est compris généralement entre 2 et 13, de préférence entre 6 et 10.

Le réglage du pH de la composition peut être obtenu à l'aide d'un agent alcalin, tel que, par exemple, l'ammoniaque, les amines organiques comme la monoéthanolamine, la diéthanolamine, la triéthanolamine, la 1,3-propanediamine et le 2-amino-2-methyl-1-propanol, un carbonate ou bicarbonate alcalin ou d'ammonium, un carbonate organique tel que le carbonate de guanidine, un hydroxyde alcalin tel que la soude, ou bien à l'aide d'un agent acidifiant tel que par exemple l'acide chlorhydrique, l'acide acétique, l'acide lactique, l'acide oxalique ou l'acide borique.

La composition réductrice comprend généralement un ou plusieurs solvants cosmétiquement acceptables, choisis de préférence parmi l'eau, les alcools en C₁-C₆, de préférence les alcanols tels que l'éthanol, le propanol et l'isopropanol, les polyols tels que le propylène glycol, le pentanediol et la glycérine, l'alcool benzylique, les éthers de polyols, les esters en C₂-C₆, la N-méthylpyrrolidone (NMP), et les cétones en C₃-C₆.

Dans le but d'améliorer les propriétés cosmétiques des fibres capillaires ou encore d'atténuer ou d'éviter leur dégradation, la composition réductrice utilisée selon l'invention peut également comprendre un ou plusieurs additifs cosmétiques.

Ce ou ces additifs sont généralement choisis parmi les silicones volatiles ou non, linéaires ou cycliques, les polymères cationiques, les peptides et leurs dérivés, les hydrolysats de protéines, les cires, les agents de gonflement et de pénétration ou permettant de renforcer l'efficacité du ou des réducteurs tels que le mélange SiO₂/polydiméthylsiloxane, le diméthylisosorbitol, l'urée et ses dérivés, les agents tensioactifs anioniques, cationiques, non ioniques, amphotères ou zwittérioniques, les agents antichute, les agents antipelliculaires, les épaississants naturels ou synthétiques, associatifs ou non, les agents de suspension, les agents séquestrants, les agents opacifiants, les colorants, les filtres solaires, les vitamines ou provitamines, les huiles minérales, végétales ou synthétiques, ainsi que les parfums et les conservateurs, et leurs mélanges.

La composition réductrice utilisée dans le procédé selon l'invention peut se présenter sous la forme d'une lotion, épaissie ou non, d'une crème, d'un gel ou d'une mousse.

L'application de la composition réductrice telle que définie précédemment constitue donc la première étape du procédé selon l'invention.

Après l'application de la composition réductrice, on peut laisser poser ladite composition, généralement pendant 5 à 60 minutes, de préférence 5 à 30 minutes, éventuellement sous casque.

Comme expliqué précédemment, le procédé selon l'invention comprend, après l'étape d'application de la composition réductrice, une éventuelle étape de rinçage puis une étape d'élévation de la température des fibres capillaires, au moyen d'un fer chauffant, à une température au moins égale à 60°C.

Au sens de la présente invention, on entend par fer un dispositif de chauffage des fibres capillaires par contact.

L'extrémité du fer venant en contact avec les cheveux peut avoir différentes formes. Elle peut notamment présenter une surface plane ; on parle dans ce cas de fer plat. Elle peut également présenter une surface arrondie ; on parle dans ce cas de fer rond.

L'application du fer peut se faire par touches séparées successives de quelques secondes, ou par déplacement ou glissement progressif le long des mèches.

A titre d'exemple de fer utilisable dans le procédé selon l'invention, on peut citer tous types de fer plats ou ronds et, en particulier, de manière non limitative, ceux décrits dans les brevets US 4 103 145, US 4 308 878, US 5 983 903, US 5 957 140, US 5 494 058 et US 5 046 516.

De préférence, la température des fibres capillaires est élevée jusqu'à une température comprise entre 60°C et 250°C, mieux entre 120°C et 220°C.

Selon un mode de réalisation préféré, les fibres capillaires ne sont pas rincées avant l'étape d'élévation de la température.

Le procédé selon l'invention peut également comprendre une étape supplémentaire de pré-séchage partiel des fibres capillaires avant l'étape d'élévation de la température, de manière à éviter d'importants dégagements de vapeurs qui pourraient brûler les mains du coiffeur et le cuir chevelu du sujet. Cette étape de pré-séchage peut se faire par exemple au moyen d'un séchoir, d'un casque, ou bien encore par séchage libre. Le procédé selon l'invention ne comprend pas d'étape de fixation par application d'une composition oxydante.

La présente invention a également pour objet l'utilisation du procédé tel que décrit précédemment pour modifier de manière durable la forme de la chevelure avec une faible dégradation de la couleur des cheveux et/ou avec une faible dégradation des fibres capillaires.

La présente invention est illustrée par les exemples suivants.

### Exemples

On met en oeuvre le procédé de traitement des fibres capillaires selon l'invention en utilisant une composition réductrice.

Les compositions réductrices testées sont les suivantes.

| | |
|---|---|
| Composition réductrice 1 Sulfite d'éthanolamine | 1,5g |
| MEXOMERE PO | 2,5g |
| Agent de pH | qs pH 7 |
| Eau déminéralisée | qsp 100g |

Les essais sont réalisés sur des cheveux colorés naturellement frisés.

Sur la tête, une composition réductrice telle que décrite précédemment est appliquée. On laisse poser la composition pendant 5 minutes.

On effectue ensuite un pré-séchage partiel des cheveux au moyen d'un sèche-cheveux.

Puis on passe au fer plat chauffé à 180°C.

On constate au final un bon toucher de la fibre capillaire, une maîtrise du volume, un bon respect de la couleur et une bonne rémanence dans le temps

## Revendications

1. Procédé de traitement des fibres capillaires **caractérisé en ce qu'**il comprend les étapes suivantes :
- application sur les fibres capillaires d'une composition réductrice contenant au moins un agent réducteur choisis parmi les sulfites ou les bisulfites, ladite composition réductrice ne contenant pas de céramides, puis
- élévation de la température des fibres capillaires, au moyen d'un fer chauffant, à une température au moins égale à 60°C, l'élévation de la température étant effectuée avant ou après le rinçage éventuel des fibres capillaires, ledit procédé ne comprenant pas d'étape de fixation par application d'une composition oxydente.

2. Procédé selon la revendication 1 **caractérisé en ce que** la composition réductrice ne contient pas d'acide dithiodiglycolique ou l'un de ses sels.

3. Procédé selon les revendications 1 à 2 **caractérisé en ce que** la température est élevée jusqu'à une température comprise entre 60°C et 250°C, de préférence entre 120°C et 220°C.

4. Procédé selon la revendication 3 **caractérisé en ce que** la composition réductrice est appliquée sur des fibres capillaires humides et propres.

5. Procédé selon l'une quelconque des revendications précédentes **caractérisé en ce que**, après l'application de la composition réductrice, on laisse poser ladite composition réductrice avant l'application du fer.

6. Procédé selon l'une quelconque des revendications précédentes **caractérisé en ce que** les fibres capillaires ne sont pas rincées avant l'étape d'élévation de la température.

7. Procédé selon l'une quelconque des revendications précédentes **caractérisé en ce qu'**il comprend une étape de pré-séchage partiel des fibres capillaires avant l'étape d'élévation de la température.

8. Procédé selon les revendications 1 à 7 **caractérisé en ce que** les sulfites ou bisulfites son choisis parmi les sulfites ou bisulfites des métaux alcalins ou alcalino-terreux, ou d'ammonium ou d'alcanolamines.

9. Procédé selon l'une quelconque des revendications précédentes **caractérisé en ce que** le ou les agents réducteurs représentent de 0,1 à 30%, de préférence de 0,5 et 20%, mieux de 1 à 10% en poids par rapport au poids total de la composition réductrice.

10. Procédé selon l'une quelconque des revendications précédentes **caractérisé en ce que** le pH de la composition réductrice est compris entre 2 et 13, de préférence entre 6 et 10.

11. Procédé selon l'une quelconque des revendications précédentes **caractérisé en ce que** la composition réductrice comprend un ou plusieurs solvants cosmétiquement acceptables choisis parmi l'eau, les alcools en C₁-C₆, de préférence les alcanols tels que l'éthanol, le propanol et l'isopropanol, les polyols tels que le propylène glycol, le pentanediol et la glycérine, l'alcool benzylique, les éthers de polyols, les esters en C₂-C₆, la N-méthylpyrrolidone (NMP), et les cétones en C₃-C₆.

12. Procédé selon l'une quelconque des revendications précédentes **caractérisé en ce que** la composition réductrice comprend un ou plusieurs additifs cosmétiques choisis parmi les silicones volatiles ou non, linéaires ou cycliques, les polymères cationiques, les peptides et leurs dérivés, les hydrolysats de protéines, les cires, les agents de gonflement et de pénétration ou permettant de renforcer l'efficacité du ou des réducteurs tels que le mélange SiO₂/polydiméthylsiloxane, le diméthylisosorbitol, l'urée et ses dérivés, les agents tensioactifs anioniques, cationiques, non ioniques, amphotères ou zwittérioniques, les agents antichute, les agents antipelliculaires, les épaississants naturels ou synthétiques, associatifs ou non, les agents de suspension, les agents séquestrants, les agents opacifiants, les colorants, les filtres solaires, les vitamines ou provitamines, les huiles minérales, végétales ou synthétiques, ainsi que les parfums et les conservateurs, et leurs mélanges.

13. Procédé selon l'une quelconque des revendications précédentes **caractérisé en ce que** la composition réductrice se présente sous la forme d'une lotion, épaissie ou non, d'une crème ou d'un gel ou d'une mousse.

14. Utilisation du procédé selon l'une quelconque des revendications précédentes pour modifier de manière durable la forme de la chevelure avec une faible dégradation de la couleur des cheveux et une faible dégradation des fibres.

## Patentansprüche

1. Verfahren zur Behandlung von Haarfasern, **dadurch gekennzeichnet, dass** es die folgenden Schritte umfasst:
- Aufbringen einer reduzierenden Zusammensetzung, die mindestens ein Reduktionsmittel enthält, auf die Haarfasern, wobei das Reduktionsmittel aus Sulfiten oder Bisulfiten ausgewählt ist und die reduzierende Zusammensetzung keine Ceramide enthält, dann
- Erhöhen der Temperatur der Haarfasern mit Hilfe eines Heizeisens auf eine Temperatur mindestens gleich 60°C, wobei die Temperaturerhöhung vor oder nach fakultativem Spülen der Haarfasern durchgeführt wird, wobei das Verfahren keinen Schritt des Fixierens durch Aufbringen einer oxidierenden Zusammensetzung umfasst.

2. Verfahren nach Anspruch 1, **dadurch gekennzeichnet, dass** die reduzierende Zusammensetzung keine Dithiodiglykolsäure oder ein Salz davon enthält.

3. Verfahren nach den Ansprüchen 1 bis 2, **dadurch gekennzeichnet, dass** man die Temperatur auf eine Temperatur zwischen 60°C und 250°C, vorzugsweise zwischen 120°C und 220°C, erhöht.

4. Verfahren nach Anspruch 3, **dadurch gekennzeichnet, dass** man die reduzierende Zusammensetzung auf feuchte und saubere Haarfasern aufbringt.

5. Verfahren nach einem der vorhergehenden Ansprüche, **dadurch gekennzeichnet, dass** man die reduzierende Zusammensetzung nach dem Aufbringen vor der Applikation des Eisens einwirken lässt.

6. Verfahren nach einem der vorhergehenden Ansprüche, **dadurch gekennzeichnet, dass** man die Haarfasern vor dem Temperaturerhöhungsschritt nicht spült.

7. Verfahren nach einem der vorhergehenden Ansprüche, **dadurch gekennzeichnet, dass** es einen Schritt der teilweisen Vortrocknung der Haarfasern vor dem Temperaturerhöhungsschritt umfasst.

8. Verfahren nach Ansprüchen 1 bis 7, **dadurch gekennzeichnet, dass** die Sulfite oder Bisulfite aus Sulfiten oder Bisulfiten von Alkali- oder Erdalkalimetallen oder von Ammonium oder von Alkanolaminen ausgewählt werden.

9. Verfahren nach einem der vorhergehenden Ansprüche, **dadurch gekennzeichnet, dass** das Reduktionsmittel bzw. die Reduktionsmittel 0,1 bis 30 Gew.-%, vorzugsweise 0,5 bis 20 Gew.-% und noch besser 1 bis 10 Gew.-%, bezogen auf das Gesamtgewicht der reduzierenden Zusammensetzung, ausmacht bzw. ausmachen.

10. Verfahren nach einem der vorhergehenden Ansprüche, **dadurch gekennzeichnet, dass** der pH-Wert der reduzierenden Zusammensetzung zwischen 2 und 13 und vorzugsweise zwischen 6 und 10 liegt.

11. Verfahren nach einem der vorhergehenden Ansprüche, **dadurch gekennzeichnet, dass** die reduzierende Zusammensetzung ein oder mehrere kosmetisch unbedenkliche Lösungsmittel, die aus Wasser, C₁-C₆-Alkoholen, vorzugsweise Alkanolen wie Ethanol, Propanol und Isopropanol, Polyolen wie Propylenglykol, Pentandiol und Glycerin, Benzylalkohol, Polyolethern, C₂-C₆-Estern, N-Methylpyrrolidon (NMP) und C₃-C₆-Ketonen ausgewählt sind, umfasst.

12. Verfahren nach einem der vorhergehenden Ansprüche, **dadurch gekennzeichnet, dass** die reduzierende Zusammensetzung ein oder mehrere kosmetische Additive, die aus linearen oder cyclischen, flüchtigen oder nichtflüchtigen Silikonen, kationischen Polymeren, Peptiden und Derivaten davon, Proteinhydrolysaten, Wachsen, Quellmitteln, Penetriermitteln, Mitteln zur Verstärkung der Wirksamkeit des Reduktionsmittels bzw. der Reduktionsmittel wie die SiO₂/Polydimethylsiloxan-Mischung, Dimethylisosorbitol, Harnstoff und Derivaten davon, anionischen, kationischen, nichtionischen, amphoteren oder zwitterionischen Tensiden, Mitteln zur Bekämpfung von Haarverlust, Antischuppenmitteln, natürlichen oder synthetischen, assoziativen oder nicht assoziativen Verdickern, Suspendiermitteln, Sequestriermitteln, Trübungsmitteln, Farbmitteln, Lichtschutzmitteln, Vitaminen oder Provitaminen, mineralischen, pflanzlichen oder synthetischen Ölen sowie Duftstoffen und Konservierungsmitteln und Mischungen davon ausgewählt sind, umfasst.

13. Verfahren nach einem der vorhergehenden Ansprüche, **dadurch gekennzeichnet, dass** die reduzierende Zusammensetzung in Form einer verdickten oder unverdickten Lotion, einer Creme oder eines Gels oder eines Schaums vorliegt.

14. Verwendung des Verfahrens nach einem der vorhergehenden Ansprüche zur dauerhaften Modifizierung der Form des Haars mit geringer Degradation der Farbe der Haare und geringer Degradation der Fasern.

## Claims

1. Process for treating hair fibres, **characterized in that** it comprises the following steps:
- applying to the hair fibres a reducing composition containing at least one reducing agent chosen from sulfites or bisulfites, the said reducing composition not containing any ceramides, then
- raising the temperature of the hair fibres, by means of a heating iron, to a temperature at least equal to 60°C, the raising of the temperature being performed before or after optional rinsing of the hair fibres, the said process not comprising any fixing step by application of an oxidizing composition.

2. Process according to Claim 1, **characterized in that** the reducing composition does not contain any dithiodiglycolic acid or a salt thereof.

3. Process according to Claims 1 to 2, **characterized in that** the temperature is raised up to a temperature of between 60°C and 250°C, preferably between 120°C and 220°C.

4. Process according to Claim 3, **characterized in that** the reducing composition is applied to clean, wet hair fibres.

5. Process according to any one of the preceding claims, **characterized in that**, after applying the reducing composition, the said reducing composition is left in place before applying the iron.

6. Process according to any one of the preceding claims, **characterized in that** the hair fibres are not rinsed before the temperature raising step.

7. Process according to any one of the preceding claims, **characterized in that** it comprises a step of partial predrying of the hair fibres before the temperature raising step.

8. Process according to Claims 1 to 7, **characterized in that** the sulfites or bisulfites are chosen from sulfites or bisulfites of alkali metals or alkaline-earth metals, or of ammonium or of alkanolamines.

9. Process according to any one of the preceding claims, **characterized in that** the reducing agent(s) represent from 0.1 to 30%, preferably from 0.5 to 20%, better from 1 to 10% by weight relative to the total weight of the reducing composition.

10. Process according to any one of the preceding claims, **characterized in that** the pH of the reducing composition is between 2 and 13, preferably between 6 and 10.

11. Process according to any one of the preceding claims, **characterized in that** the reducing composition comprises one or more cosmetically acceptable solvents chosen from water, C₁-C₆ alcohols, preferably alkanols such as ethanol, propanol and isopropanol, polyols such as propylene glycol, pentanediol and glycerol, benzyl alcohol, polyol ethers, C₂-C₆ esters, N-methylpyrrolidone (NMP) and C₃-C₆ ketones.

12. Process according to any one of the preceding claims, **characterized in that** the reducing composition comprises one or more cosmetic additives chosen from linear or cyclic, volatile or non-volatile silicones, cationic polymers, peptides and derivatives thereof, protein hydrolysates, waxes, swelling agents and penetrants or agents for reinforcing the efficacy of the reducing agent(s) such as the SiO₂/polydimethylsiloxane mixture, dimethylisosorbitol, urea and derivatives thereof, anionic, cationic, nonionic, amphoteric or zwitterionic surfactants, hair-loss counteractants, antidandruff agents, natural or synthetic, associative or non-associative thickeners, suspension agents, sequestrants, opacifiers, dyes, sunscreens, vitamins or provitamins, mineral, plant or synthetic oils, and also fragrances and preserving agents, and mixtures thereof.

13. Process according to any one of the preceding claims, **characterized in that** the reducing composition is in the form of a thickened or unthickened lotion, a cream or a gel or a mousse.

14. Use of the process according to any one of the preceding claims, for durably modifying the shape of the head of hair with a slight degradation of the colour of the hair and a slight degradation of the fibres.
